(19) Europäisches Patentamt
European Patent Office
Office européen des brevets

(11) EP 0 633 258 B1

(12) **EUROPÄISCHE PATENTSCHRIFT**

(45) Veröffentlichungstag und Bekanntmachung des Hinweises auf die Patenterteilung:
**10.11.1999 Patentblatt 1999/45**

(51) Int Cl.$^6$: **C07D 317/46**, C07C 403/24

(21) Anmeldenummer: **94109921.0**

(22) Anmeldetag: **27.06.1994**

# (54) Verbessertes Verfahren zur Herstellung von Astaxanthin, neue Zwischenprodukte hierfür sowie ein Verfahren zu deren Herstellung

Improved process for the production of astaxanthin, new intermediates therefor and a process for their production

Procédé amélioré de production d'astaxantin, nouveaux produits intermédiares et procédé de leur préparation

(84) Benannte Vertragsstaaten:
**CH DE FR GB LI NL**

(30) Priorität: **05.07.1993 DE 4322277**

(43) Veröffentlichungstag der Anmeldung:
**11.01.1995 Patentblatt 1995/02**

(73) Patentinhaber: **BASF Aktiengesellschaft
67063 Ludwigshafen (DE)**

(72) Erfinder:
- **Ernst, Hansgeorg, Dr.
D-67346 Speyer (DE)**
- **Dobler, Walter, Dr.
D-69126 Heidelberg (DE)**
- **Paust, Joachim, Dr.
D-67141 Neuhofen (DE)**
- **Rheude, Udo, Dr.
D-67166 Otterstadt (DE)**

(56) Entgegenhaltungen:
**EP-A- 0 005 749**
**EP-A- 0 101 597**
**EP-A- 0 455 119**

- **CHEMICAL ABSTRACTS, Band 96, Nr. 11, 15. M rz 1982, Columbus, Ohio, USA E. WIDMER et al. "Technical procedures for the synthesis of carotenoids and related compounds from 6-oxoisophorone. II. A novel concept for the synthesis of (3RS,3'RS)-astaxanthin" Seite 613, Spalte 2, Nr. 85 791p; & Helv. Chim. Acta 1981, 64(7),2436-46**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 633 258 B1

Printed by Jouve, 75001 PARIS (FR)

## Beschreibung

Die Erfindung betrifft Verbindungen der allgemeinen Formel I

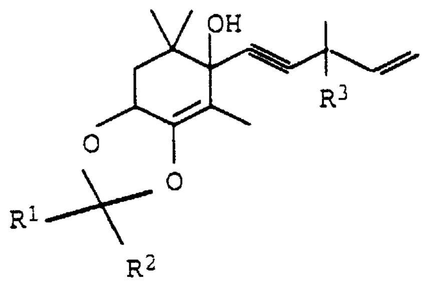

,

(I)

in der $R^1$ für H oder eine $C_1$-$C_4$-Alkylgruppe, $R^2$ für eine $C_1$-$C_4$-Alkylgruppe und $R^3$ für eine durch Hydrolyse in eine Hydroxygruppe überführbare Ether-, Silylether- oder AcetalSchutzgruppe, vorzugsweise für einen der Reste

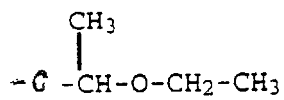

oder

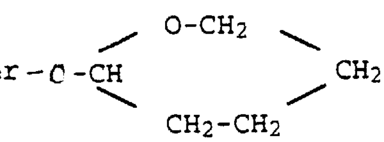

stehen,
deren Herstellung und deren Verwendung zur Herstellung von Astaxanthin sowie anderen essentiellen Astaxanthin-vorprodukten.

Das $C_{40}$-Carotinoid Astaxanthin der Formel V

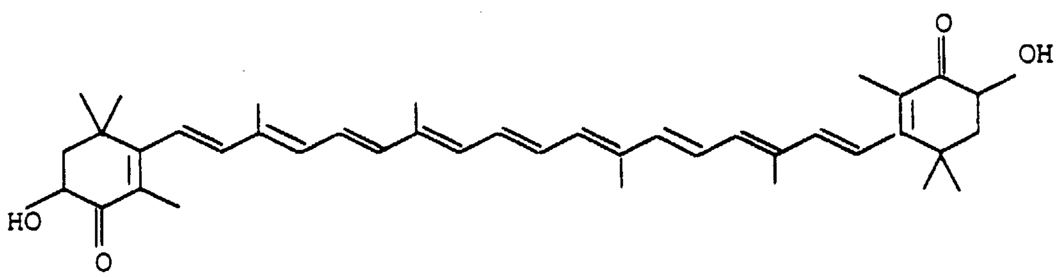

(V)

ist ein für die Fischpigmentierung begehrter Farbstoff. Die Möglichkeiten für eine Isolierung von Astaxanthin aus natürlichen Quellen, wie Algen oder Hefen sind begrenzt. Es hat daher nicht an Versuchen gefehlt, Astaxanthin synthetisch herzustellen.

Eine technisch realisierbare Synthese von Astaxanthin wird in EP 5748 sowie in Helv. Chim. Acta 64 (1981) Seiten 2436ff beschrieben. Sie verläuft nach den Syntheseschema:

$$C_9 + C_6 \rightarrow C_{15}$$

$$C_{15} + C_{10} + C_{15} \rightarrow C_{40}$$

Als $C_9$-Baustein wird jeweils 2,2,4,6,6-Pentamethyl-7,7a-dihydro-2H,6H-1,3-benzodioxol-5-on eingesetzt, das aus dem 3,4-Dihydroxy-2,6,6-trimethyl-2-cyclohexen-1-on durch Umsetzung mit Aceton oder 2,2-Dimethoxy-propan hergestellt werden kann:

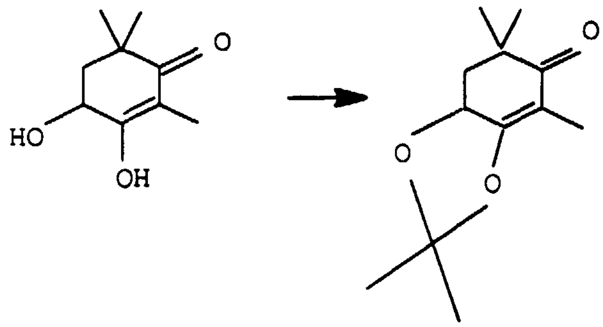

Als $C_6$-Baustein wird gemäß den genannten Literaturstellen 3-Methyl-pentenin-1-ol der Formel

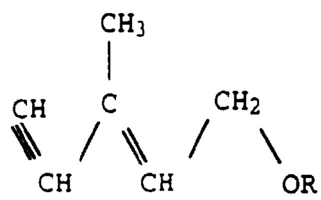

mit geschützter OH-Gruppe genannt. Als Schutzgruppen werden neben der Trialkylsilylgruppe die tert.-Butylgruppe sowie die Gruppe -$C(CH_3)_2$-O-$CH_3$ genannt. In EP 5748 wird als $C_6$-Baustein zusätzlich der Trialkylsilylether des 3-Methyl-pentenin-3-ols der Formel

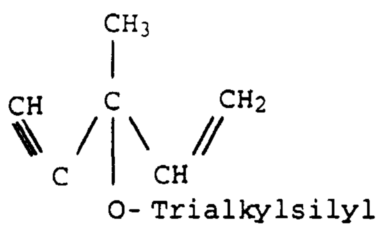

genannt. Experimentelle Beispiele für die Umsetzung dieses 3-Methyl-pentenin-3-ol-ethers mit dem $C_9$-Baustein liegen nicht vor. Schlüsselschritt der Synthese ist die Verknüpfung des $C_9$-Bausteins mit einem $C_6$-Baustein mittels einer metallorganischen Reaktion. Zur Deprotonierung des Acetylens wird entweder ein Grignardreagens in Tetrahydrofuran (THF) oder eine Butyllithiumlösung verwendet. In Helv. Chim. Acta 64 (1981), Seite 2439 werden die Grignard-Variante und die Butyllithium-Variante systematisch verglichen; dabei wird der Butyllithium-Variante eindeutig den Vorzug gegeben. Die Ausbeute an dem Kupplungsprodukt betrug hierbei 85,6 % der Theorie (d. Th.).

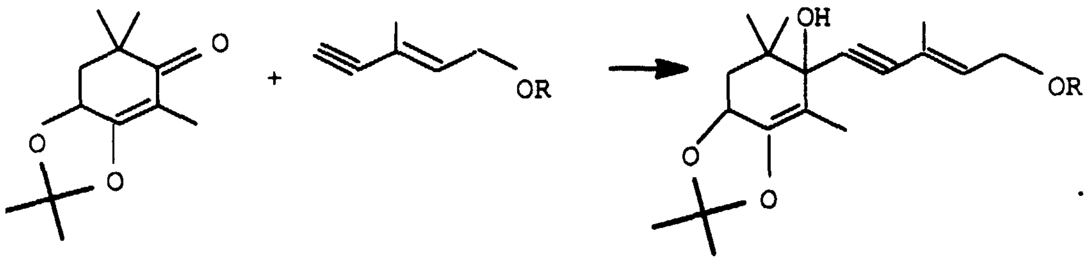

Aus diesem Kupplungsprodukt werden bei Aufarbeitung in mineralsaurem Milieu alle Schutzgruppen abgespalten unter Bildung von 6-Hydroxy-3-(3-methyl-5-hydroxy-3-penten-1-inyl)-2,4,4-trimethyl-2-cyclohexenon. Durch Reduktion der Dreifachbindung mit Zinkstaub und Essigsäure in $CH_2Cl_2$, anschließende Umsetzung mit HBr und danach mit Triphenylphosphin kann hieraus ein $C_{15}$-Triphenylphosphoniumsalz hergestellt werden, das mit 2,7-Dimethyl-2,4,6-octatrien-1,8-dial zu Astaxanthin umgesetzt werden kann.

Nachteilig an dem an sich recht guten Verfahren ist die Notwendigkeit des Arbeitens mit Butyllithium, welches sehr teuer, sehr leicht endzündlich und verfahrenstechnisch nicht einfach zu handhaben ist. Zudem liegt das Butyllithium im allgemeinen in Hexan gelöst vor, so daß man nach der Umsetzung ein Gemisch aus organischen Lösungsmitteln aufarbeiten müßte.

Auch die Verwendung eines Grignard-Reagenses ist technisch nicht sehr vorteilhaft durchführbar wegen Schwierigkeiten beim Handling der niedrigsiedenden Alkylhalogenide sowie wegen möglicher verfahrenstechnischer Probleme im Hinblick auf die Herstellung des Grignardreagenses (Anspringen der Reaktion).

Es war daher die Aufgabe der Erfindung, die bekannte Synthese von Astaxanthin so zu verbessern, daß die Nachteile des Standes der Technik bei der Verknüpfung des $C_9$-Bausteins mit einem $C_6$-Baustein vermieden werden.

Es wurde nun überraschenderweise gefunden, daß die Verknüpfung des $C_9$-Bausteins mit einem entsprechenden $C_6$-Baustein unter technisch sehr viel einfacheren Bedingungen, nämlich in einem organischen Lösungsmittel in Gegenwart von Lithiumamid anstelle von Butyllithium bzw. einem Grignard-Reagens, gelingt, wenn man als $C_6$-Baustein Derivate des 3-Methyl-pentenin-3-ols der allgemeinen Formel II verwendet.

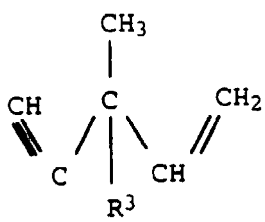

(II),

in der $R^3$ für eine durch Hydrolyse in eine Hydroxygruppe überführbare Ether-, Silylether- oder Acetal-Schutzgruppe, insbesondere einen der Reste

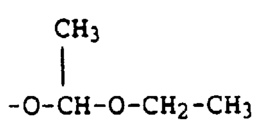

oder

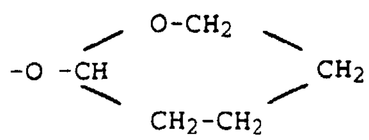

steht. Bei dieser Umsetzung bilden sich die bisher in der Literatur noch nicht beschriebenen Verbindungen der allgemeinen Formel I.

Der glatte Verlauf dieser Reaktion ist deshalb besonders überraschend, weil ausgehend von dem C6-Baustein mit der Ethergruppe in 1-Stellung der Formel

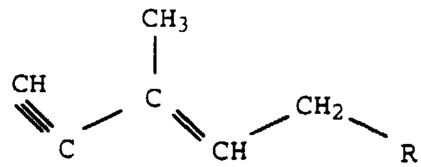 ,

in dem R für

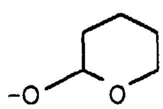 oder 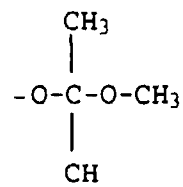

steht,
in Methyl-tert.-butylether keine Umsetzung mit dem $C_9$-Baustein beobachtet werden konnte.

Gegenstand der Erfindung ist daher auch ein Verfahren zur Herstellung der Verbindungen der allgemeinen Formel I

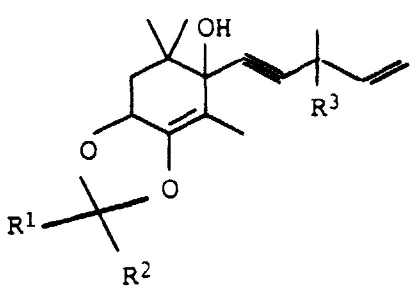 ,

(I)

in der $R^1$ für H oder eine $C_1$-$C_4$-Alkylgruppe, $R^2$ für eine$C_1$-$C_4$-Alkylgruppe und $R^3$ für eine durch Hydrolyse in eine Hydroxygruppe überführbare Ether-, Silylether- oder Acetal-Schutzgruppe stehen, das dadurch gekennzeichnet ist, daß man ein Alkenin der allgemeinen Formel II

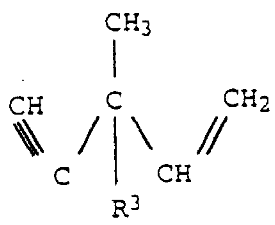

(II),

in der $R^3$ die oben angegebene Bedeutung hat, in einem inerten Lösungsmittel unter Mitverwendung von Lithiumamid mit einem Cyclohexenon der allgemeinen Formel III

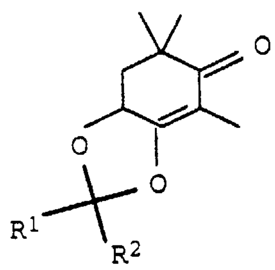

(III),

in der $R^1$ und $R^2$ die oben angegebene Bedeutung haben, umsetzt.

Als $C_9$-Bausteine werden Cyclohexenone der allgemeinen Formel III bevorzugt, in denen $R^1$ für H oder Methyl und $R^2$ für Methyl stehen. Das Cyclohexenon der Formel III mit $R^1$ = H und $R^2$ = Methyl ist in der Literatur noch nicht beschrieben. Es kann durch Umsetzen von 3,4-Dihydroxy-2,6,6-trimethyl-2-cyclohexen-1-on mit Vinylethylether erhalten werden. Die Umsetzung dieses $C_9$-Bausteines mit einem Alkenin der Formel II, in der $R^3$ für den Rest $-O-CH(CH_3)-O-CH_2-CH_3$ steht, ist technisch und ökonomisch besonders vorteilhaft, da beide Bausteine mit dem gleichen Schutzgruppenlieferanten, dem Vinylethylether, hergestellt werden. Dieses Alkenin ist bereits aus J. Org. Chem. 47 (1982) Seiten 2130 bis 2134 bekannt. Gemäß loc. cit. wird es in einer Canthaxanthin-Synthese mit Butyllithium metalliert und dann mit 2,6,6-Trimethyl-2-cyclohexenon verknüpft.

Als durch Hydrolyse in die Hydroxygruppe überführbare Schutzgruppen in den Alkeninen der allgemeinen Formel II kommen solche Schutzgruppen in Betracht, die relativ leicht in die Hydroxygruppe überführt werden können. Genannt seien beispielsweise Ethergruppen, wie

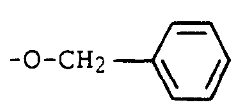

und $-O-C(CH_3)_3$,

Silylethergruppen, wie $-O-Si(CH_3)_3$ oder Acetalgruppen, wie die die α-Alkoxy-alkylethergruppen der Formeln $-O-CH_2-O-CH_3$,

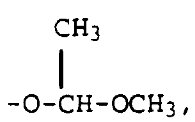 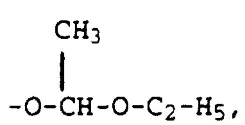 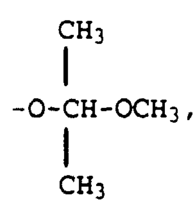

und geeignete Pyranylethergruppen, wie die Tetrahydropyranyloxygruppe und die 4-Methyl-5,6-dihydro-2H-pyranyloxy-Gruppe.

Mit besonderem Vorteil verwendet man Alkenine der Formel II, in denen $R^3$ für die Tetrahydropyranyloxygruppe

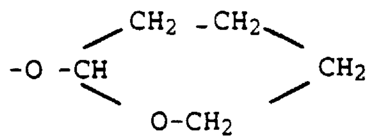

oder die α-Ethoxy-ethoxygruppe der Formel

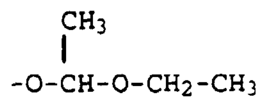

stehen.

Die Alkenine der Formel II verwendet man im allgemeinen in einem Überschuß von 0 bis 100 Mol.-%, vorzugsweise 50 bis 75 Mol.-%, bezogen auf den $C_9$-Baustein der Formel III.

Als inertes Lösungsmittel kommen bei dem erfindungsgemäßen Verfahren generell gegenüber Lithiumamid inerte Solventien in Betracht. Mit Vorteil arbeitet man in einem etherischen Lösungsmittel, wie Dialkylethern, Tetrahydrofuran oder Dioxan, insbesondere in dem mit Wasser nicht mischbaren Methyl-tert.-butylether, oder aber in dem mit Wasser nicht mischbaren Toluol.

Das Lithiumamid verwendet man im allgemeinen in Mengen von 1,0 bis 1,05, vorzugsweise etwa 1,02 Äquivalenten, bezogen auf das Alkenin der Formel II.

Zur Durchführung des Verfahrens arbeitet man im allgemeinen so, daß man festes Lithiumamid in dem inerten Lösungsmittel suspendiert, zu dieser Suspension langsam das Alkenin der Formel II addiert, wobei dieses durch das Lithiumamid deprotoniert wird. Zu der erhaltenen Suspension des Lithiumalkenins läßt man den $C_9$-Baustein der Formel III zulaufen. Nach mehrstündiger Nachreaktion hydrolysiert man durch Zugabe von Wasser. Bei Verwendung von Methyl-tert.-butylether als Lösungsmittel befindet sich das Wertprodukt der Formel I in der organischen Oberphase, was den technischen Ablauf des Verfahrens sehr vereinfacht. Es kann durch Abdestillieren des Lösungsmittels und destillatives Entfernen von eventuell überschüssigem $C_6$-Baustein in ca. 95 %iger Ausbeute isoliert werden. Der abdestillierte $C_6$-Baustein kann problemlos in die Synthese zurückgeführt werden.

Es war überraschend, daß die Umsetzung des Cyclohexenon der Formel III mit dem Alkenin der Formel II so vorteilhaft in einem inerten organischen Lösungsmittel durchgeführt werden kann, da nach dem Stand der Technik beispielsweise die Umsetzung von 6-oxo-isophoron der Formel

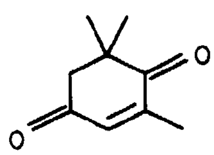

mit einem Alkenin bei Verwendung eines Lithiumamids bei Temperaturen von -40°C und in flüssigem Ammoniak durchgeführt werden mußte (vgl. Helv. Chim. Acta 65 (1982) Nr. 89, Seiten 958 - 967, insbesondere Seite 960), was technisch sehr aufwendig und teuer ist.

Die Reaktionstemperaturen für die beschriebene Verknüpfungsreaktion liegen im allgemeinen zwischen Raumtemperatur und dem Siedepunkt des eingesetzten Lösungsmittels.

Durch Schutzgruppenabspaltung in wäßrigsaurem Milieu bildet sich aus den erfindungsgemäßen Verbindungen der Formel I das 6-Hydroxy-3-(3-hydroxy-3-methyl-4-penten-1-inyl)-4,4,6-trimethyl -2-cyclohexen-1-on der Formel VI

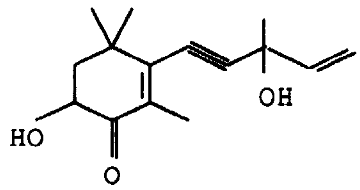

(VI)

in praktisch quantitativer Ausbeute. Dieses Alkindiol der Formel VI ist bereits aus dem Verfahren gemäß Helv. Chim. Acta 65 (1982) Seiten 671ff bekannt, wonach es trotz Verwendung von Butyllithium bei der Verknüpfung nur in einer Ausbeute von 56,2 % erhalten wurde. Das Alkindiol der Formel VI kann mit Zinkstaub und Essigsäure in einem chlorierten Kohlenwasserstoff wie Methylenchlorid oder in anderen inerten Solventien, wie Methyl-tert.-butylether oder Toluol, oder aber in Eisessig zu dem $C_{15}$-Diol der Formel IV

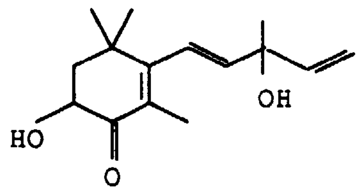

(IV)

reduziert werden. Die Reduktion des Alkindiols der Formel VI mit Zink/Essigsäure ist in der Literatur noch nicht beschrieben.

Vorzugsweise verwendet man eine etwa 20 %ige Lösung des Alkindiols der Formel VI in Methylenchlorid/Eisessig, wobei man letzteren im Verhältnis von etwa 1:2 bis 1:2,5 anwendet. Das Zink wird zweckmäßig in einer Menge von etwa 1 bis 3 Grammatomen, vorzugsweise etwa 1,3 bis 1,5 Grammatomen pro Mol Ausgangsmaterial verwendet. Die Temperaturen für diese Hydrierung liegen bei -20°C bis Raumtemperatur, vorzugsweise bei etwa 0°C.

Das so erhaltene $C_{15}$-Diol der Formel IV kann dann wie in Helv. Chim. Acta 64 (1981) Seiten 2419 bis 2446 beschrieben vorteilhaft in Astaxanthin überführt werden. Darüber hinaus wurde gefunden, daß man die Astaxanthinsynthese ausgehend von dem Alkindiol der Formel VI auch ohne Zwischenisolierung des gebildeten $C_{15}$-Diols der Formel IV, des daraus hergestellten $C_{15}$-Bromids der Formel VII sowie des daraus hergestellten $C_{15}$-Triphenylphosphoniumsalzes der Formel VIII, also praktisch in einer Eintopfreaktion, durchführen kann. Hierdurch ergeben sich weitere große Vorteile bei der Synthese von Astaxanthin gegenüber der gemäß dem Stand der Technik.

Gegenstand der Erfindung ist daher auch ein sehr vorteilhaftes Gesamtverfahren zur Herstellung von Astaxanthin der Formel V, das dadurch gekennzeichnet ist, daß man

A. ein Alkenin der allgemeinen Formel II

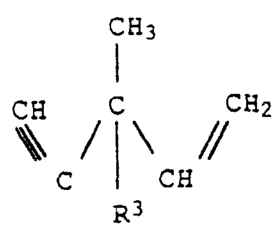

(II),

in der

$R^3$ für eine durch Hydrolyse in eine Hydroxygruppe überführbare Ether-, Silylether oder Acetal-Schutzgruppe steht, in einem inerten Lösungsmittel unter Mitverwendung von Lithiumamid mit einem Cyclohexenon der allgemeinen Formel III

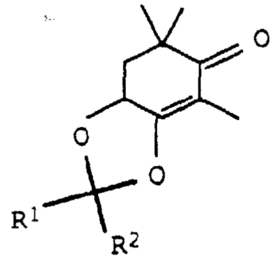

(III),

in der $R^1$ für H oder eine $C_1$-$C_4$-Alkylgruppe und $R^2$ für eine $C_1$-$C_4$-Alkylgruppe stehen, umsetzt,
B. aus der erhaltenen Verbindung der allgemeinen Formel I

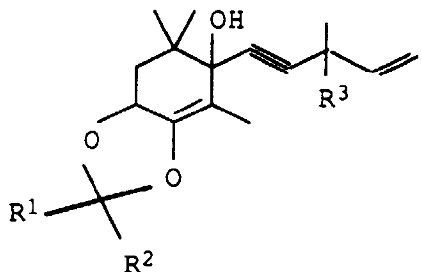

,

(I)

in wäßrig-saurem Milieu die Schutzgruppen abspaltet,
C. das erhaltene Alkindiol der Formel VI

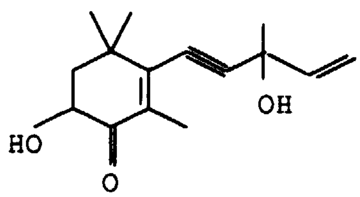

(VI)

mit Zink-Staub in Methylenchlorid/Essigsäure reduziert
D. das erhaltene $C_{15}$-Diol der Formel IV

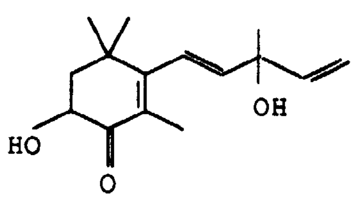

(IV)

mit Chlorwasserstoffsäure oder Bromwasserstoffsäure umsetzt,
E. das erhaltene $C_{15}$-Halogenid der Formel VII

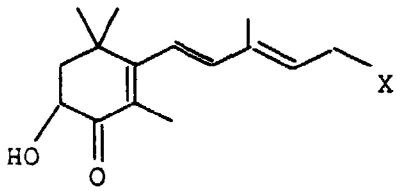

(VII),

in der X für Cl oder Br steht,
mit Triphenylphosphin umsetzt und
F. das erhaltene Triphenylphosphoniumsalz der Formel VIII

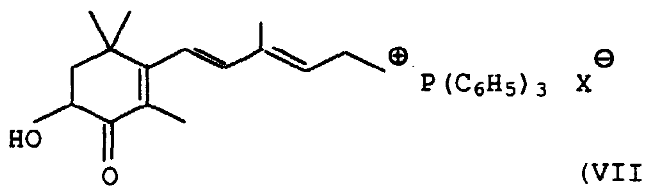

(VIII)

in einer Wittig-Reaktion mit 2,7-Dimethyl-2,4,6-octatrien-1,8-dial zu Astaxanthin umsetzt.

Beispiel 1

a. Herstellung des $C_9$-Bausteins

170 g (1,0 mol) kristallines 3,4-Dihydroxy-2,6,6-trimethyl-2-cyclohexen-1-on wurden in 500 ml Methylenchlorid suspendiert. Man gab zunächst 500 mg (2,9 mmol) p-Toluolsulfonsäure zu der Suspension zu und ließ dann innerhalb von 2 Stunden (h) bei Raumtemperatur (RT) 144 g (2,0 mol) Vinylethylether zulaufen. Anschließend rührte man 4 h bei RT nach und ließ dann 100 ml einer 5 %igen Natronlauge zulaufen. Die unten befindliche organische Phase wurde abgetrennt und die Wasserphase einmal mit 100 ml Methylenchlorid extrahiert. Die vereinigten organischen Phasen wurden mit 200 ml Wasser gewaschen und am Rotationsverdampfer eingeengt. Der Rückstand wurde unter stark vermindertem Druck (Ölpumpe) getrocknet. Man erhielt 2,4,6,6-Tetramethyl-7,7a-dihydro-6H-benzo[1,3]-dioxol-5-on als nach Dünnschichtchromatographie (DC) reines und nach Gaschromatographie (GC) annähernd reines gelbes Öl.

Das Rohprodukt wurde durch Destillation gereinigt (Übergang bei 90°C/0,1 mbar).

Die Ausbeute betrug 185 g entsprechend 94,4 % der Theorie (d.Th.).

b. Herstellung des $C_{15}$-Bausteins der allgemeinen Formel I

2,0 Liter Methyl-tert.-butylether (MTB) wurden vorgelegt. Dazu gab man 118 g (5,13 mol) festes Lithiumamid und rührte die farblose Suspension 30 Minuten (min) bei +50°C nach. Anschließend ließ man 840 g (5,0 mol) 3-(1-Ethoxyethyloxy)-3-methyl-1-penten-4-in innnerhalb von 30 min zulaufen und rührte 2 h bei +50°C nach. Man kühlte auf +25 - 30°C ab und ließ dann innerhalb von 15 min 558 g (2,85 mol) des $C_9$-Bausteins 2,4,6,6-Tetramethyl-7,7a-dihydro-6H-benzo[1,3]dioxol-5-on zulaufen. Man rührte 1,5 h bei RT nach und ließ den Ansatz dann innerhalb von 15 min in einen zweiten Kessel mit 1,5 1 Wasser laufen. Die Phasen wurden 10 min verrührt. Die Wasserphase (unten) wurde abgetrennt. Die organische Phase wurde dreimal mit je 500 ml Wasser gewaschen und am Rotationsverdampfer bei +50°C Badtemperatur bei einem Druck bis zu 150 mbar eingeengt.

Rotationsverdampfer-Rückstand: 1435 g

Überschüssiges 3-(1-Ethoxyethyloxy)-3-methyl-1-penten-4-in wurde mittels Sambay-Destillation (Manteltemperatur 110°C, 2 - 3 mbar, Übergangstemperatur 40 - 45°C) abgetrennt. Sambay-Ablauf: 1023 g 5-[3-(1-Ethoxyethoxy)-3-methyl-pent-4-en-1-inyl]-2,4,6,6-tetramethyl-5,6,7,7a-tetrahydro-benzo[1,3]-dioxol-5-ol mit einer Reinheit von 95 %.

c. Herstellung von 6-Hydroxy-3-(3-hydroxy-3-methyl-4-penten -1-inyl)-2,4,4-trimethyl-2-cyclohexen-1-on (VI)

603 g (1,65 mol) gemäß 1b erhaltenen tertiären Alkohols der Formel I (Sambay-Ablauf) wurden in 1400 ml $CH_2Cl_2$ gelöst. Hierzu ließ man 500 ml Wasser und anschließend 250 ml einer 30 %igen $H_2SO_4$ zulaufen und rührte über Nacht bei RT nach. Die organische Phase (unten) wurde abgetrennt und je einmal mit 500 ml einer 5

%igen $NaHCO_3$-Lösung und 500 ml Wasser gewaschen.

Die organische Phase wurde am Rotationsverdampfer eingeengt und der ölige Rückstand unter stark vermindertem Druck getrocknet.

Auswaage: 408 g 6-Hydroxy-3-(3-hydroxy-3-methyl-4-penten1-inyl)-2,4,4-trimethyl-2-cyclohexen-1-on entsprechend einer quantitativen Rohausbeute.

d. Herstellung des Triphenylphosphoniumsalzes der Formel VIII

248 g (1 mol) des gemäß Beispiel Ic hergestellten rohen Alkindiols der Formel VI wurden in 1000 ml Methylenchlorid gelöst. Man kühlte auf 0°C ab und ließ 180 g (3,0 mol) Essigsäure zulaufen. Danach gab man bei 0°C im Abstand von 15 min jeweils 11 g Zinkpulver zu (insgesamt 88 g Zink entsprechend 1,35 g-Atom). Nach der letzten Zinkzugabe wurde 45 min bei 0°C nachgerührt. Das entstandene Zinkacetat wurde abfiltriert und der Filterkuchen zweimal mit je 250 ml Methylenchlorid gewaschen. Zum Filtrat ließ man innerhalb von 15 min bei 0°C 258 g einer 47 %igen wäßrigen Lösung von HBr (1,50 mol HBr) zulaufen und rührte 20 min bei 0°C nach. Dann ließ man 900 ml Wasser zulaufen und trennte die organische Phase (unten) ab. Die Wasserphase wurde einmal mit 150 ml Methylenchlorid gewaschen. Die vereinigten organischen Phasen wurden mit 900 ml Wasser versetzt. Nach Zugabe von 47 g festem $NaHCO_3$ wurden die Phasen einige min verrührt. Die Unterphase wurde abgetrennt, mit 900 ml Wasser gewaschen und mit 8 ml 1,2-Epoxybutan versetzt. Unter Kühlung auf $\leq$ +10°C gab man innerhalb von ca. 15 min portionsweise 262 g (1,0 mol) festes Triphenylphosphin zu, ließ innerhalb von ca. 30 min auf RT kommen und gab nochmals 8 ml 1,2-Epoxy-butan zu.

Anschließend wurde bei Normaldruck Methylenchlorid unter gleichzeitigem Zulauf von MTB abdestilliert, bis eine Übergangstemperatur von +55°C erreicht war.

Die Triphenylphosphoniumsalzsuspension wurde auf RT abgekühlt, 30 min bei RT nachgerührt und abgesaugt. Der Filterkuchen wurde zweimal mit je 800 ml MTB gewaschen und über Nacht im $N_2$-Strom getrocknet.

Auswaage: 419 g (73 % d.Th.)

Beispiel 2

Herstellung von Astaxanthin aus dem Alkindiol der Formel VI ohne Isolierung des Triphenylphosphoniumsalzes der Formel .VIII.

100 g rohes Alkindiol der Formel VI (Reinheit nach GC ca. 86 %) wurden in 400 ml Methylenchlorid gelöst. Man kühlte auf 0°C ab und ließ 72 g Essigsäure zulaufen. Bei 0°C gab man im Abstand von 15 min jeweils 4,4 g Zinkpulver (insgesamt 35,2 g Zink) zu. Nach der letzten Zinkzugabe wurde 45 min bei 0°C nachgerührt. Das entstandene Zinkacetat wurde abfiltriert und der Filterkuchen zweimal mit je 70 ml Methylenchlorid gewaschen. Das Filtrat wurde zweimal mit je 300 ml Wasser gewaschen und innerhalb von 30 min bei 0°C zu 104 g einer 47 %igen wäßrigen HBr getropft. Man rührte 30 min bei 0°C nach, ließ 360 ml Wasser zulaufen und trennte die organische Phase ab. Die Wasserphase wurde einmal mit 50 ml Methylenchlorid extrahiert. Die vereinigten organischen Phasen wurden mit 360 ml Wasser versetzt. Nach Zugabe von 47 g festem $NaHCO_3$ wurden die Phasen kurz miteinander verrührt. Die Unterphase wurde abgetrennt, mit 360 ml Wasser gewaschen und mit 3 ml 1,2-Epoxy-butan versetzt. Unter Kühlung auf Temperaturen $\leq$ +10°C gab man 105 g Triphenylphosphin zu und rührte 18 h bei RT nach. Danach gab man 22,8 g (0,139 mol) des $C_{10}$-Dialdehyds 2,7-Dimethyl-2,4,6-octatrien-1,8-dial zu, kühlte auf 0°C ab und ließ bei 0°C 57,5 g einer 30 %igen methanolischen Natriummethylatlösung zulaufen. Man rührte 3 h bei 0°C nach und gab dann 500 ml Wasser zu. Die organische Phase wurde abgetrennt und die Wasserphase zweimal mit je 100 ml Methylenchlorid extrahiert. Die vereinigten organischen Phasen wurden einmal mit 400 ml Wasser gewaschen. Bei Normaldruck wurde Methylenchlorid unter gleichzeitigem Zulauf von Methanol abdestilliert, bis eine Übergangstemperatur von +65°C erreicht war. Die Suspension wurde 15 h unter Rückfluß zum Sieden erhitzt und dann auf 0°C abgekühlt. Das erhaltene Kristallisat wurde abfiltriert, mit Methanol und Heptan gewaschen und dann in 500 ml Methylenchlorid aufgenommen. Der Lösungsmitteltausch gegen Methanol wurde wie oben beschrieben wiederholt. Nach Trocknung des Filterkuchens erhielt man 63 g (76,0 % d. Th.) Astaxanthin mit einer Reinheit von 98,2 % nach HPLC.

## Patentansprüche

1. Verbindungen der allgemeinen Formel I

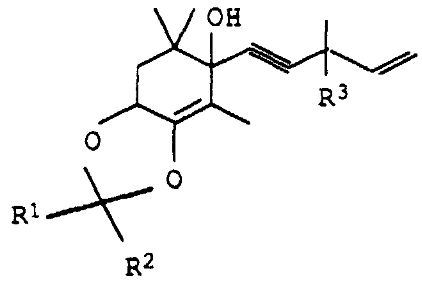

,

(I)

in der

$R^1$ für H oder eine $C_1$-$C_4$-Alkylgruppe,
$R^2$ für eine $C_1$-$C_4$-Alkylgruppe und
$R^3$ für eine durch Hydrolyse in eine Hydroxygruppe überführbare Ether-, Silylether oder Acetal-Schutzgruppe steht.

**2.** Verbindungen der allgemeinen Formel I gemäß Anspruch 1, in denen $R^1$ und $R^2$ die in Anspruch 1 angegebene Bedeutung haben und $R^3$ für einen der Reste

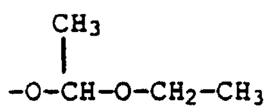

oder

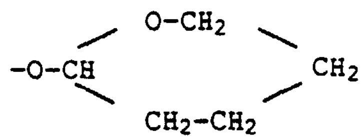

steht.

**3.** Verfahren zur Herstellung der Verbindungen der allgemeinen Formel I

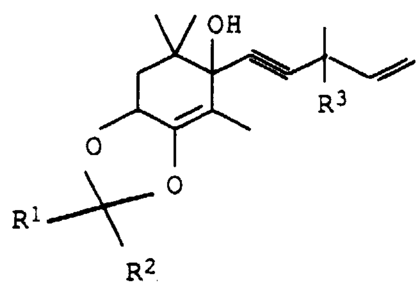

,

(I)

in der

$R^1$ für H oder eine $C_1$-$C_4$-Alkylgruppe,
$R^2$ für eine $C_1$-$C_4$-Alkylgruppe und
$R^3$ für eine durch Hydrolyse in eine Hydroxygruppe überführbare Ether-, Silylether- oder Acetal-Schutzgruppe

stehen, das dadurch gekennzeichnet ist, daß man ein Alkenin der allgemeinen Formel II

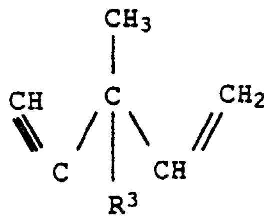

(II),

in der $R^3$ die oben angegebene Bedeutung hat in einem inerten Lösungsmittel unter Mitverwendung von Lithiumamid mit einem Cyclohexenon der allgemeinen Formel III

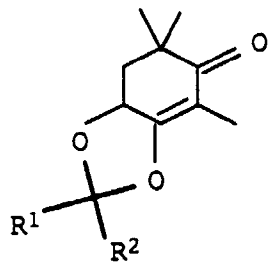

(III),

in der $R^1$ und $R^2$ die oben angegebene Bedeutung haben,
umsetzt.

4. Verfahren gemäß Anspruch 3, dadurch gekennzeichnet, daß man die Umsetzung in Methyl-tert.-butylether als inertem Lösungsmittel durchführt.

5. Verfahren gemäß Anspruch 3, dadurch gekennzeichnet, daß man das Alkenin der allgemeinen Formel II in einem Überschuß von 0 bis 100 mol.-%, bezogen auf das Cyclohexenon der Formel III einsetzt.

6. Verfahren gemäß Anspruch 3, dadurch gekennzeichnet, daß man die Umsetzung bei Temperaturen von Raumtemperatur bis zum Siedepunkt des verwendeten Lösungsmittels durchführt.

7. Verwendung der Verbindungen gemaß Anspruch 1 zur Herstellung von 6-Hydroxy-3-(3-hydroxy-3-methyl-1,4-pentadien-1-yl)-2,4,4-trimethyl-2-cyclohexen-1-on der Formel IV

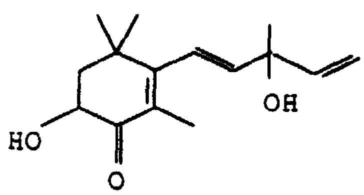

(IV)

8. Verwendung der Verbindungen gemäß Anspruch 1 zur Herstellung von Astaxanthin der Formel V

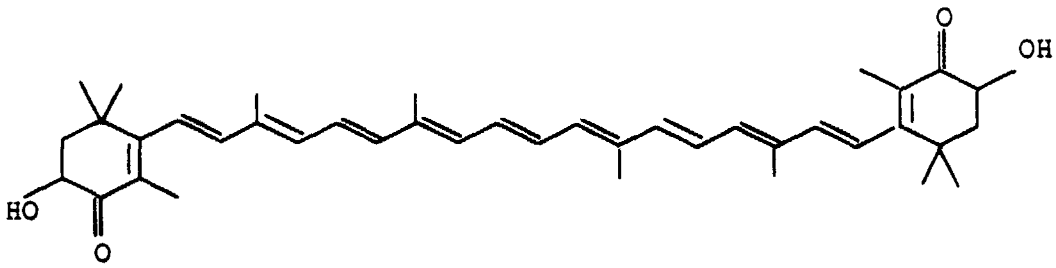

(V)

**9.** Verfahren zur Herstellung von Astaxanthin der Formel V, dadurch gekennzeichnet, daß man

A. ein Alkenin der allgemeinen Formel II

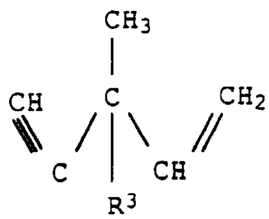

(II),

in der
$R^3$ für eine durch Hydrolyse in eine Hydroxygruppe überführbare Ether-, Silylether- oder Acetal-Schutzgruppe steht, in einem inerten Lösungsmittel unter Mitverwendung von Lithiumamid mit einem Cyclohexenon der allgemeinen Formel III

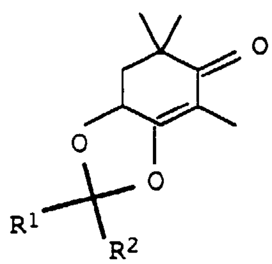

(III),

in der

$R^1$ für H oder eine $C_1$-$C_4$-Alkylgruppe und
$R^2$ für eine $C_1$-$C_4$-Alkylgruppe stehen,

umsetzt,
B. aus dem erhaltenen tertiären Alkohol der allgemeinen Formel I

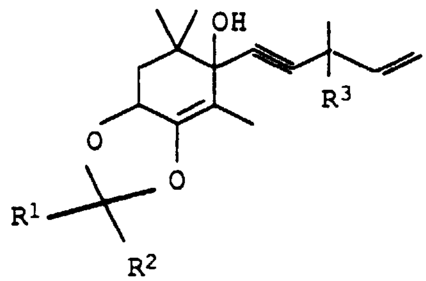

,

(I)

in wäßrig-saurem Milieu die Schutzgruppen abspaltet,
C. das erhaltene Alkindiol der Formel VI

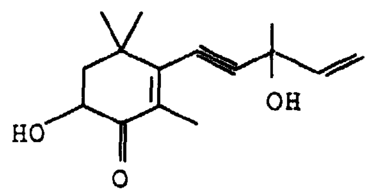

(VI)

mit Zink-Staub in Methylenchlorid/Essigsäure reduziert
D. das erhaltene $C_{15}$-Diol der Formel IV

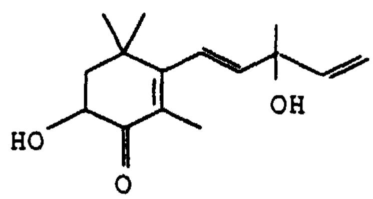

(IV)

mit Chlorwasserstoffsäure oder Bromwasserstoffsäure umsetzt,
E. das erhaltene Halogenid der Formel VII

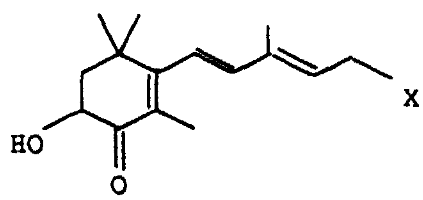

(VII),

in der X für Cl oder Br steht,
mit Triphenylphosphin umsetzt und
F. das erhaltene Triphenylphosphoniumsalz der Formel VIII

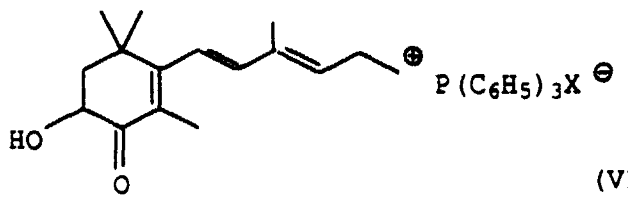

(VIII),

in der X für Cl oder Br steht,
in einer Wittig-Reaktion mit 2,7-Dimethyl-2,4,6-octatrien-1,8-dial zu Astaxanthin umsetzt.

**10.** Verfahren zur Herstellung von Astaxanthin der Formel V gemäß Anspruch 9, dadurch gekennzeichnet, daß man die Stufen C., D., E. und F. ohne Isolierung der Zwischenprodukte der Formel IV, VII und VIII durchführt.

**Claims**

**1.** A compound of the formula I

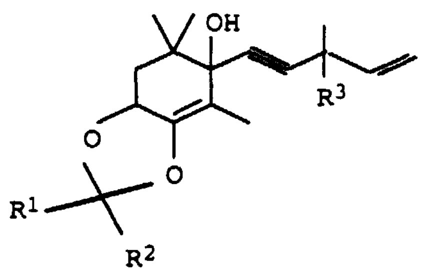

,

(I)

where

$R^1$ is H or $C_1$-$C_4$-alkyl,
$R^2$ is $C_1$-$C_4$-alkyl and
$R^3$ is an ether, silyl ether or acetal protective group which can be converted into a hydroxyl group by hydrolysis.

**2.** A compound of the formula I as claimed in claim 1, where $R^1$ and $R^2$ have the meanings stated in claim 1, and $R^3$ is one of the radicals

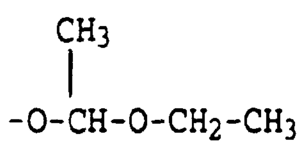

or

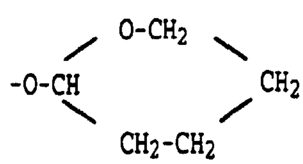

.

**3.** A process for preparing a compound of the formula I

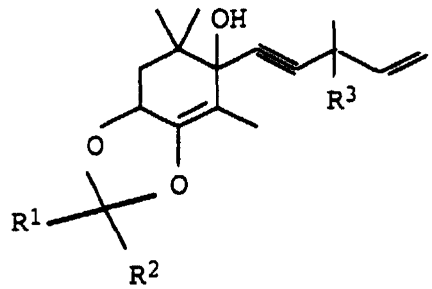

,

(I)

where

$R^1$ is H or $C_1$-$C_4$-alkyl,
$R^2$ is $C_1$-$C_4$-alkyl and
$R^3$ is an ether, silyl ether or acetal protective group which can be converted into a hydroxyl group by hydrolysis, which comprises reacting an alkenyne of the formula II

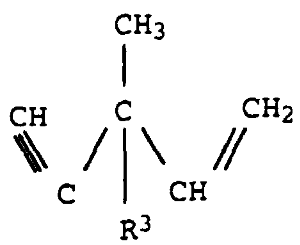

(II),

where $R^3$ has the abovementioned meanings, in an inert solvent in the presence of lithium amide with a cyclohexenone of the formula III

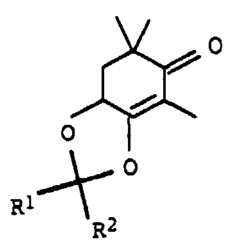

(III),

where $R^1$ and $R^2$ have the abovementioned meanings.

**4.** A process as claimed in claim 3, wherein the reaction is carried out in methyl tert-butyl ether as inert solvent.

**5.** A process as claimed in claim 3, wherein the alkenyne of the formula II is employed in an excess of from 0 to 100 mol % based on the cyclohexenone of the formula III.

**6.** A process as claimed in claim 3, wherein the reaction is carried out at from room temperature to the boiling point of the solvent used.

**7.** The use of a compound as claimed in claim 1 for preparing 6-hydroxy-3-(3-hydroxy-3-methyl-1,4-pentadien-1-yl)-2,4,4-trimethyl-2-cyclohexen-1-one of the formula IV

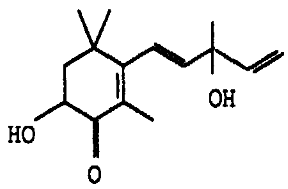

(IV).

**8.** The use of a compound as claimed in claim 1 for preparing astaxanthin of the formula V

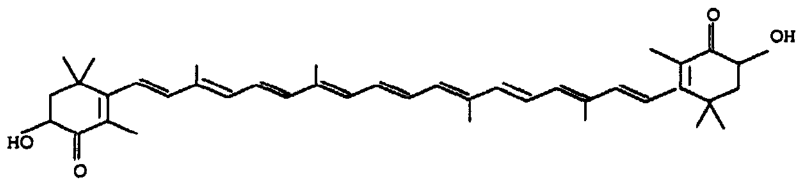

(V).

**9.** A process for preparing astaxanthin of the formula V, which comprises

A. reacting an alkenyne of the formula II

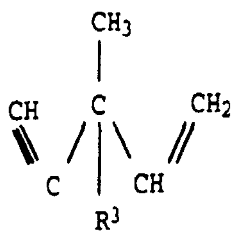

(II),

where

$R^3$ is an ether, silyl ether or acetal protective group which can be converted into a hydroxyl group by hydrolysis, in an inert solvent in the presence of lithium amide with a cyclohexenone of the formula III

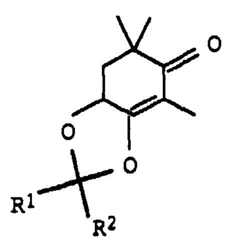

(III),

where

$R^1$ is H or $C_1$-$C_4$-alkyl and
$R^2$ is $C_1$-$C_4$-alkyl,

B. eliminating from the resulting tertiary alcohol of the formula I

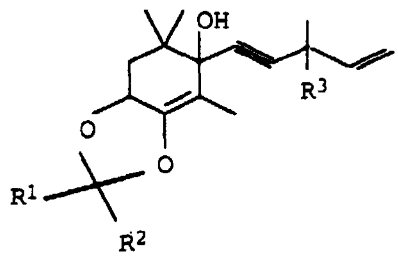

,

(I)

the protective groups in aqueous acidic medium,

C. reducing the resulting alkynediol of the formula VI

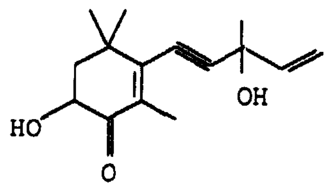

(VI)

with zinc dust in methylene chloride/acetic acid,

D. reacting the resulting $C_{15}$-diol of the formula IV

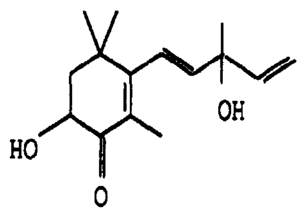

(IV)

with hydrochloric acid or hydrobromic acid,

E. reacting the resulting halide of the formula VII

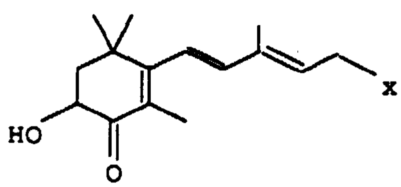

(VII)

where X is Cl or Br,

with triphenylphosphine, and

F. subjecting the resulting triphenylphosphonium salt of the formula VIII

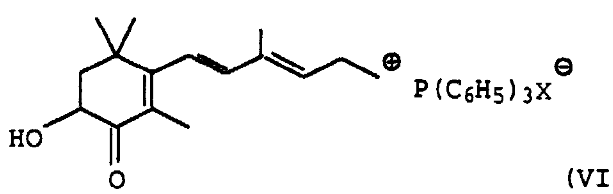

(VIII)

where X is Cl or Br,

to a Wittig reaction with 2,7-dimethyl-2,4,6-octatrienedial to give astaxanthin.

**10.** A process for preparing astaxanthin of the formula V as claimed in claim 9, wherein stages C., D., E. and F. are carried out without isolating the intermediates of the formula IV, VII and VIII.

## Revendications

**1.** Composés de formule générale I

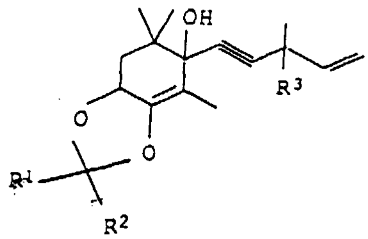

(I),

dans laquelle

$R^1$ représente H ou un groupe alkyle en C1-C4,

$R^2$ représente un groupe alkyle en C1-C4 et

$R^3$ représente un groupe protecteur éther, éther silylique ou acétal convertible par hydrolyse en un groupe hydroxy.

**2.** Composés de formule générale I selon la revendication 1, dans laquelle $R^1$ et $R^2$ ont les significations indiquées dans la revendication 1 et $R^3$ représente l'un des groupes

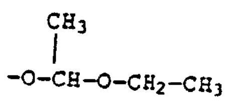

ou

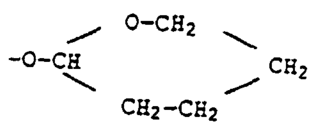

**3.** Procédé de préparation des composés de formule générale I

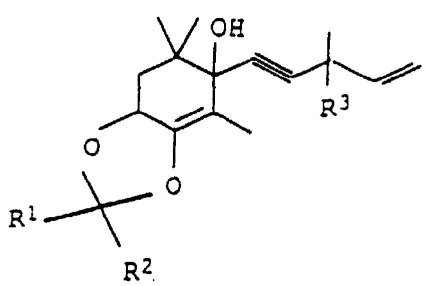

(I),

dans laquelle

$R^1$ représente H ou un groupe alkyle en C1-C4,

$R^2$ représente un groupe alkyle en C1-C4 et

$R^3$ représente un groupe protecteur éther, éther silylique ou acétal convertible en un groupe hydroxy par hydrolyse,

caractérisé par le fait que l'on fait réagir un alcényne de formule générale II

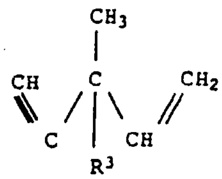

(II),

dans laquelle $R^3$ a les significations indiquées ci-dessus, dans un solvant inerte, et avec utilisation conjointe d'amidure de lithium, avec une cyclohexanone de formule générale III

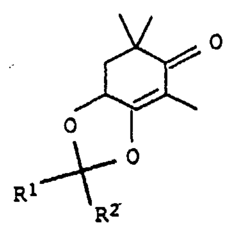

(III),

dans laquelle $R^1$ et $R^2$ ont les significations indiquées ci-dessus.

4. Procédé selon la revendication 3, caractérisé par le fait que la réaction est effectuée dans l'oxyde de méthyle et de tert-butyle, solvant inerte.

5. Procédé selon la revendication 3, caractérisé par le fait que l'on met en oeuvre l'alcényne de formule générale II en excès de 0 à 100 mol % par rapport à la cyclohexénone de formule III.

6. Procédé selon la revendication 3, caractérisé par le fait que l'on effectue la réaction à des températures allant de la température ambiante jusqu'au point d'ébullition du solvant utilisé.

7. Utilisation des composés selon la revendication 1, pour la préparation de la 6-hydroxy-3-(3-hydroxy-3-méthyl-1,4-pentadién-1-yl)-2,4,4-triméthyl-2-cyclohexén-1-one de formule IV

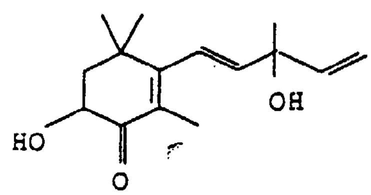

(IV)

8. Utilisation des composés selon la revendication 1 pour la préparation de l'astaxanthine de formule V

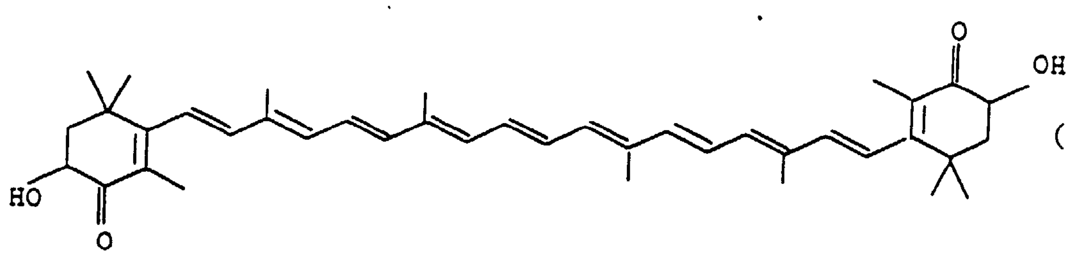

(V)

**9.** Procédé de préparation de l'astaxanthine de formule V, caractérisé par le fait que

A. on fait réagir un alcényne de formule générale II

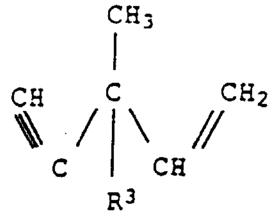

(II),

dans laquelle
$R^3$ représente un groupe protecteur éther, éther silylique ou acétal convertible par hydrolyse en un groupe hydroxy, dans un solvant inerte, avec utilisation conjointe d'amidure de lithium, avec une cyclohexénone de formule générale III

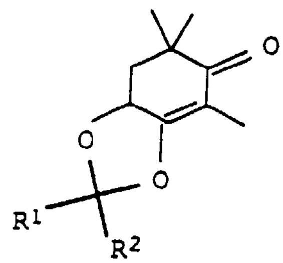

(III),

dans laquelle

$R^1$ représente H ou un groupe alkyle en C1-C4 et

$R^2$ représente un groupe alkyle en C1-C4,

B. de l'alcool tertiaire ainsi obtenu, répondant à la formule générale I

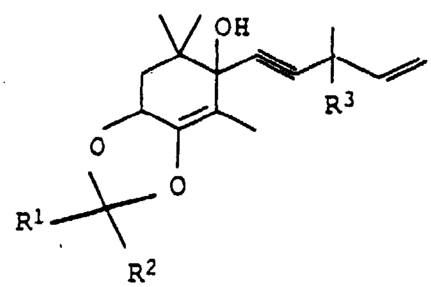

(I),

on scinde les groupes protecteurs en milieu aqueux acide.
C. ce qui donne l'alcynediol de formule VI

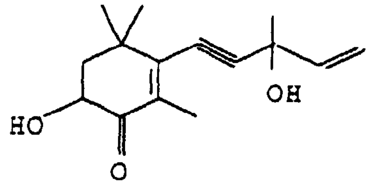

(VI)

qu'on réduit par le zinc en poudre dans un mélange chlorure de méthylène/acide acétique,
D, ce qui donne le diol en C15 de formule IV

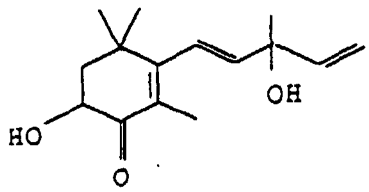

(IV)

qu'on fait réagir avec l'acide chlorhydrique ou l'acide bromhydrique,
E. ce qui donne l'halogénure de formule VII

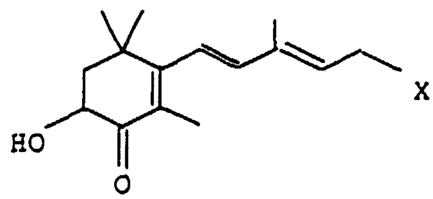

(VII),

dans laquelle X représente Cl ou Br,
qu'on fait réagir avec la triphénylphosphine,
F. ce qui donne le sel de triphénylphosphonium de formule VIII

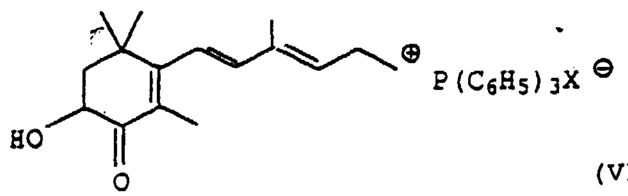

(VIII),

dans laquelle X représente Cl ou Br,
qu'on convertit en astaxanthine par une réaction de Wittig avec le 2,7-diméthyl-2,4,6-octatriène-1,8-dial.

**10.** Procédé pour la préparation de l'astaxanthine de formule V selon la revendication 9, caractérisé par le fait que l'on parcourt les stades opératoires C, D, E et F sans isoler les produits intermédiaires de formules IV, VII et VIII.